# EUROPEAN PATENT APPLICATION

(11) **EP 3 984 578 A2**
(43) Date of publication of application: **20.04.2022**
(21) Application number: 20827923.2
(22) Date of filing: 17.06.2020
(51) Int. Cl.: A61M 5/32, A61M 5/50

(54) **DISPOSABLE SAFETY SYRINGE NEEDLE AND SYRINGE DEVICE INCLUDING SAME**

(30) Priority: 17.06.2019 KR 20190071241
(71) Applicant: Noh, Wook Rea, Suwon-si, Gyeonggi-do 16305 (KR)
(72) Inventor: Noh, Wook Rea, Suwon-si, Gyeonggi-do 16305 (KR)
(74) Representative: Isarpatent
(86) International application number: PCT/KR2020/007807
(87) International publication number: WO 2020/256388

(57) **Abstract**

The present invention relates to a disposable safety syringe needle and a syringe device including same, and can prevent reuse and an accident in safety, in which injury can be sustained by the needle. In addition, the present invention comprises: an upper part (10) to which an elastic member (40) is fixed and coupled and in which a needle member (50) is inserted and fixed; a lower part (20) which is coupled to one end of the upper part (10), and which includes one or more catching parts (240); and a safety part (30) which is coupled to one end of the lower part (20), and which includes a catching protrusion (320), wherein the safety part (30) is inserted into the lower part (20) toward the upper part (10) so that the elastic member (40) is compressed, and then the catching protrusion (320) of the safety part (30) is coupled to the one or more catching parts (240) by means of the stretching force of the elastic member (40) so as to disable reuse.

## Description

### TECHNICAL FIELD

The present disclosure relates to a disposable safety syringe needle and a syringe device including the same and, more particularly, to a disposable safety syringe needle configured to prevent reuse thereof as well as accidents in which the needle causes injury and a syringe device including the same.

### BACKGROUND ART

A syringe is a medical instrument used to inject a medicine or a medical fluid into the body or extract blood or the like. The syringe is generally used in the treatment of diseases.

In general, a syringe includes a barrel in which a vacuum is created to remove or inject a liquid, a plunger movable up and down within the barrel, and a metal needle obliquely recessed and connected to a needle hole provided in the barrel.

In the modern medicine, it is ruled in the Medical Service Act that a needle portion directly linked to the risk of infection should be used only once, irrespective of whether or not a syringe is successfully put in position to inject a medicine into a patient.

In addition, recently, disposable needles having a variety of shapes capable of preventing reuse thereof have been developed.

However, such a disposable needle is exposed to the outside, so that a third person may be infected by the needle contaminated with patient blood, which is problematic.

Background-art technologies for overcoming such problems include Korean Patent Application Publication No. 10-2018-0108773 (hereinafter, referred to as Document 1), Korean Patent No. 10-0879498 (hereinafter, referred to as Document 2), Korean Patent No. 10-1852329 (hereinafter, referred to as Document 3), and Korean Patent No. 10-1858456 (hereinafter, referred to as Document 4).

Document 1 relates to a safety needle including a needle base, a needle, and a safety cap. The safety needle may be prevented from being exposed while being carried or used, and a used safety needle or a non-used safety needle may be easily identifiable.

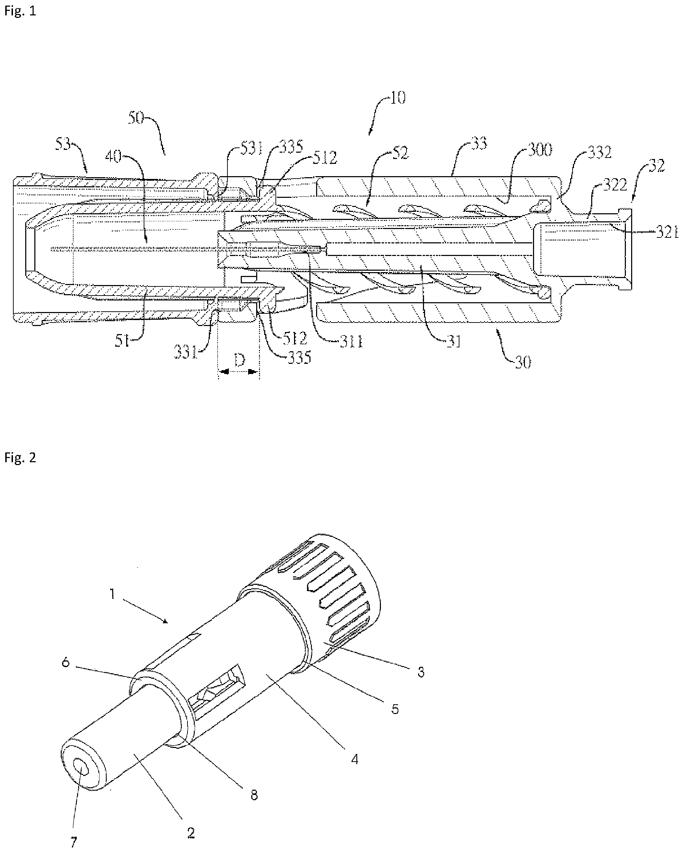

As illustrated in FIG. 1, the safety needle of Patent 1 has a structure in which the needle is protruded outward by rotational force, compressive force, and repulsive force caused by a spiral elastic member surrounding the outer circumferential surface of a needle connecting segment provided within an annular space.

In this structure, when only a small amount of force is applied to the safety needle, the needle may be easily protruded from the needle cap by the spiral elastic member, and thus an external cap is necessarily included. Thus, such a safety needle may be problematic in terms of lack of user convenience and efficiency in use.

In addition, in the safety needle of Document 1, a user may intentionally manipulate a guide block, due to the spiral elastic member, thereby causing a problem in terms of the possibility of reuse.

Document 2 relates to a safety needle assembly in which a spring positioned within a housing moves a shield in the direction of the distal end to cover a needle, a locking member additionally provided within the housing is moved simultaneously with the shield during injection, and the shield is automatically locked in a position in which a sharp distal end of a needle cannular is concealed.

As illustrated in FIG. 2, the safety needle assembly of Document 2 has a complicated structure. That is, in the protruded needle, as a locking protrusion having a sharp font surface slides toward a position formed between a flat surface on the top portion of a lip and a blocking surface on the bottom portion of the lip, the shield is locked. Thus, there may be a problem in terms of lack of user convenience.

Document 3 relates to a safety device for a pre-filled syringe and a syringe device adapted to prevent accidental puncture wounds or injuries that could otherwise be caused by a needle before, during, or after injection of a medicine or a drug contained within a pre-filled syringe.

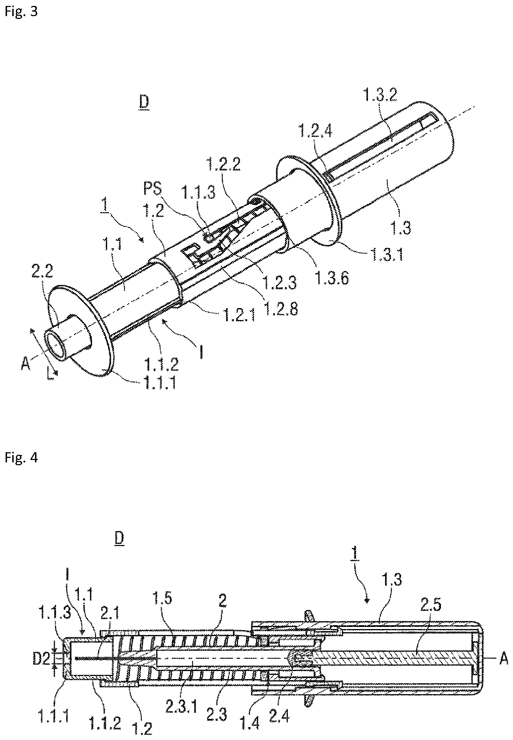

As illustrated in FIG. 3, the safety device for a pre-filled syringe includes a hollow support body for accommodating a pre-filled syringe, a hollow needle cover part capable of sliding with respect to the support body, and a guide means for guiding movement of the cover with respect to the support body. However, it is difficult for a guide pin to move smoothly within the support body, so that the body of the user may be injured.

In addition, in the safety device for a pre-filled syringe, a flexible arm and the guide pin are formed of an elastic plastic material. When the thickness of the flexible arm is insufficient, the flexible arm may be broken. Then, the needle may remain protruding from the skin instead of entering the skin, thereby causing a problem in that infection may be caused by the needle.

Document 4 relates to a needle cover part for a safety device, a safety device, and a syringe device adapted to prevent accidental puncture wounds that would otherwise be caused by a needle before, during, or after injection of a medicine or a drug contained within a pre-filled syringe.

As illustrated in FIG. 4, in the needle cover part for a safety device, a needle protrudes in response to a needle cover being introduced into the support body. After use, the needle cover is protruded outwardly of the support body by a spring, so that the needle is introduced. After use, when the needle cover is not fixed and force is applied to the spring, the needle may protrude. Thus, a user may be secondarily infected by the needle, which is problematic.

### Background Art Document

(Document 1) Korean Patent Application Publication No. 10-2018-0108773
(Document 2) Korean Patent No. 10-0879498
(Document 3) Korean Patent No. 10-1852329
(Document 4) Korean Patent No. 10-1858456

### DISCLOSURE

### Technical Problem

Accordingly, the present disclosure has been made in consideration of the above-described problems occurring in the related art, and an objective of the present disclosure is to prevent a contaminated needle from protruding from a safety holder part after a disposable safety syringe needle is used, thereby preventing a user from being secondarily infected by the contaminated needle without a protective cap.

Another objective of the present disclosure is to provide a simple configuration in which, after the safety holder part is moved down or up by an elastic part, the safety holder part is fixed by a fixing part so as not to move down again, thereby realizing a reuse prevention function for preventing reuse.

Another objective of the present disclosure is to allow a user to visually determine from the outside whether or not the disposable safety syringe needle is used.

Another objective of the present disclosure is to allow the disposable safety syringe needle to be coupled to a syringe or an automatic syringe for use thereof.

### Technical Solution

In order to realize at least one of the above described objectives, a disposable safety syringe needle according to the present disclosure may include: a top part (10) configured such that an elastic member (40) is coupled and fixed to the top part (10) and a needle member (50) is introduced into and fixed to the top part (10); a bottom part (20) coupled to one end of the top part (10), and including one or more latch portions (240); and a safety part (30) coupled to one end of the bottom part (20) and including a latch protrusion (320). After the safety part (30) is introduced into the bottom part (20) in a direction of the top part (10) so that the elastic member (40) is compressed, the latch protrusion (320) of the safety part (30) may be coupled to the one or more latch portions (240) due to tension of the elastic member (40), thereby making reuse of the disposable safety syringe needle impossible.

The top part (10) may include: a first housing (110) with open one and the other ends; a first flat portion (130) provided on an inner portion of the first housing (110) and including a flat surface; and a first support portion (140) and a second support portion (150) protruding from one side of the first flat portion (130) .

The elastic member (40) may be coupled and fixed to the first support portion (140), and the needle member (50) may be introduced into and fixed to the second support portion (150).

The bottom part (20) may include a hollow cylindrical second housing (210) coupled to one end of the first housing (110) and one or more latch portions (240) provided on outer peripheral portions of the second housing (210).

The safety part (30) may include a hollow cylindrical third housing (310) coupled to one end of the second housing (210) and a latch protrusion (320) protruding from one end of the third housing (310) .

The second support portion (150) including the open one and the other ends may be configured such that a diameter thereof decreases from one end to the other end thereof.

The latch portions (240) may include: a latch indicator (241) provided in an outer circumferential portion of a second housing (210) to allow whether or not the disposable safety syringe needle is used to be identified; a third support portion (242) protruding from one side of the latch indicator (241); a latch stepped portion (243) provided on one end of the third support portion (242), protruding into the second housing (210), and being inclined; and a latch recess (244) provided between the latch indicator (241) and the third support portion (242), such that the latch protrusion (320) of the safety part (30) is fixed by compression force of the elastic member (40).

In addition, the disposable safety syringe needle according to the present disclosure may further include a sealing part (60) to prevent the sterilized disposable safety syringe needle from being contaminated, wherein the sealing part (60) is removable from the disposable safety syringe needle.

The disposable safety syringe needle according to the present disclosure is coupled to a syringe device, such as a syringe or an automatic syringe, for use thereof.

### Advantageous Effects

In the disposable safety syringe needle according to the present disclosure, the contaminated needle member does not protrude from the safety holder part after the disposable safety syringe needle is used, thereby preventing a user from being secondarily infected by the contaminated needle member without a protective cap.

In addition, the present disclosure provides a simple configuration in which, after the safety holder part is moved down or up by the elastic part, the safety holder part is fixed by the fixing part so as not to move down again, thereby realizing a reuse prevention function for preventing reuse. Thus, tissues such as the blood vessel are not excessively injured.

Furthermore, according to the present disclosure, it is possible to visually determine from the outside whether or not the disposable safety syringe needle is used.

In addition, according to the present disclosure, the disposable safety syringe needle may be easily coupled to a syringe or an automatic syringe for use thereof.

### DESCRIPTION OF DRAWINGS

FIGS. 1 to 4 illustrate background-art Documents of the present disclosure;
FIG. 5 is a perspective view illustrating a disposable safety syringe needle according to the present disclosure;
FIG. 6 is an exploded perspective view illustrating the disposable safety syringe needle according to the present disclosure;
FIG. 7 is a top perspective view illustrating the disposable safety syringe needle according to the present disclosure;
FIG. 8 is a top rear perspective view illustrating the disposable safety syringe needle according to the present disclosure;
FIG. 9 is a top front view illustrating the disposable safety syringe needle according to the present disclosure;
FIG. 10 is a bottom perspective view illustrating the disposable safety syringe needle according to the present disclosure;
FIG. 11 is a perspective view illustrating the safety part according to the present disclosure;
FIG. 12 is a cross-sectional view illustrating the disposable safety syringe needle according to the present disclosure before use thereof;
FIG. 13 is a cross-sectional view illustrating the disposable safety syringe needle according to the present disclosure during use thereof;
FIG. 14 is a cross-sectional view illustrating the disposable safety syringe needle according to the present disclosure after use thereof;
FIG. 15 is a perspective view illustrating another form of the disposable safety syringe needle according to the present disclosure; and
FIG. 16 is a perspective view illustrating a syringe device to which a disposable safety syringe needle according to the present disclosure is coupled.

### <Description of Reference Numerals of Drawings>

1: disposable safety syringe needle, 2: syringe device,
10: top part, 110: first housing, 120: first guide, 121: first guide protrusion, 130: first flat portion, 140: first support portion, 150: second support portion, 160: first hole, 170: first coupling portion, 180: second hole,
20: bottom part, 210: second housing, 220: second coupling portion, 230: third coupling portion, 240: latch portion, 241: latch indicator, 242: third support portion, 243: latch stepped portion, 244: latch recess,
30: safety part, 310: third housing, 320: latch protrusion, 330: third hole,
40: elastic member,
50: needle member,
60: sealing part, 61: first sealing portion, 62: second sealing portion

### BEST MODE

Hereinafter, embodiments of the present disclosure will be described in detail.

Objectives, features, and advantages of the present disclosure will be apparent from the following embodiments.

The present disclosure should not be construed as being limited to the embodiments to be disclosed herein and may be embodied in a variety of different forms. The following embodiments will be provided for illustrative purposes to fully convey the concept of the present disclosure to those having ordinary knowledge in the technical field, and should be interpreted as including all modifications, equivalents, and substitutions without departing from the technical idea and scope of the present disclosure.

Therefore, the present disclosure is not limited by the following embodiments but should be interpreted as including all modifications without departing from the technical idea and scope of the present disclosure. That is, those having ordinary knowledge in the technical field to which the present disclosure pertains can make various modifications and variations of the present disclosure by adding, changing, deleting, or supplementing elements without departing from the principle of the present disclosure described in the Claims, and such modifications and variations should be included within the scope of the present disclosure.

Although the present disclosure may be modified variously and have a variety of embodiments, specific embodiments will be illustrated in the drawings and described in detail. In the drawings, the sizes of the elements and the relative sizes of the elements may be exaggerated for a better understanding of the present disclosure. In addition, the shapes of the elements illustrated in the drawings may be changed more or less due to changes in fabrication process or the like.

Therefore, the following embodiments should not be limited to the shapes illustrated in the drawings unless expressly so stated herein but should be understood as including some variations.

In the meantime, several embodiments of the present disclosure may be coupled to other embodiments unless explicitly stated to the contrary. In particular, any preferable or advantageous features may be combined with any other feature or features. That is, various aspects, features, embodiments, or implementations of the present disclosure may be used either alone or in various combinations.

It should be understood that each of terms used herein is to describe a specific embodiment but is not to be limited by the Claims. Unless otherwise specified, all terms including technical and scientific terms used herein have the same meaning as that commonly understood by those having ordinary knowledge in the art. As used herein, singular forms are intended to include plural forms unless the context clearly indicates otherwise.

In the description of the present disclosure, detailed descriptions of well-known functions and components incorporated herein will be omitted when it is determined that the description may make the subject matter of the present disclosure rather unclear.

### <First Embodiment>

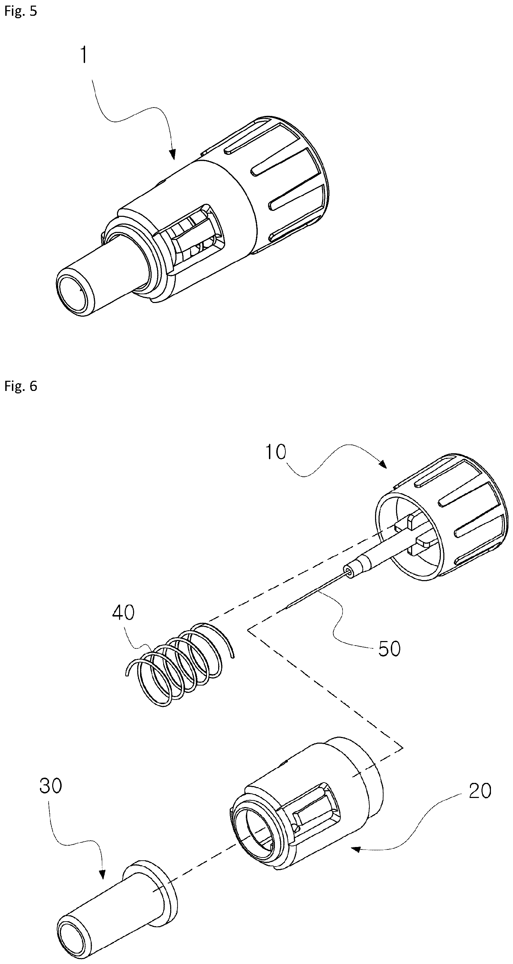

As illustrated in FIG. 5, a disposable safety syringe needle 1 may be formed of a metal or a synthetic resin. The disposable safety syringe needle 1 generally includes a top part 10, a bottom part 20, a safety part 30, an elastic member 40, and a needle member 50.

In particular, the top part 10, the bottom part 20, and the safety part 30 may be formed of a synthetic resin. Particularly, the top part 10, the bottom part 20, and the safety part 30 may be formed of, but is not limited to, polyvinyl chloride (PVC), polyethylene (PE), or polypropylene (PP).

The PVC is advantageous in terms of non-toxicity, as well as superior weather resistant and chemical stability.

The PE is advantageous in terms of not being discolored even when exposed to the sunlight, being a relatively stable material, and having high thermal resistance and durability.

The PP is advantageous in terms of having superior transparency, tensile strength, thermal resistance, chemical resistance compared to the PE and being non-harmful to the human.

The needle member 50 may be formed of plastic or metal.

When the needle member 50 is formed of a plastic, the needle member 50 is elastic and flexible. Thus, there is an advantage in that, in the case of the injection of a Ringer solution, the needle member 50 is not released from the blood vessel even when a patient moves.

In addition, when the needle member 50 is formed of a metal, the needle member 50 may be particularly formed of a stainless steel. Advantageously, the stainless steel does not contain a heavy metal or does not produce a toxic substance, and has superior durability and thermal resistance.

The elastic member 40 is formed of a metal, and may be formed of the same material as the needle member 50.

First, as illustrated in FIGS. 6 to 9, the top part 10 includes a first housing 110 with open one and the other ends and a first coupling portion 170 provided on the other end of the first housing 110 to be coupled to a syringe device 2.

In addition, a thread (not shown) is provided on the inner portion of the other end of the first housing 110. When the disposable safety syringe needle 1 is coupled to the syringe device 2, the disposable safety syringe needle 1 is not released from the syringe device 2.

In particular, since the thread is provided on the inner portion of the other end of the first housing 110, the disposable safety syringe needle 1 is tightly coupled to the syringe device 2. Thus, a seal is provided between the disposable safety syringe needle 1 and the syringe device 2, thereby advantageously preventing a contained medicine from leaking.

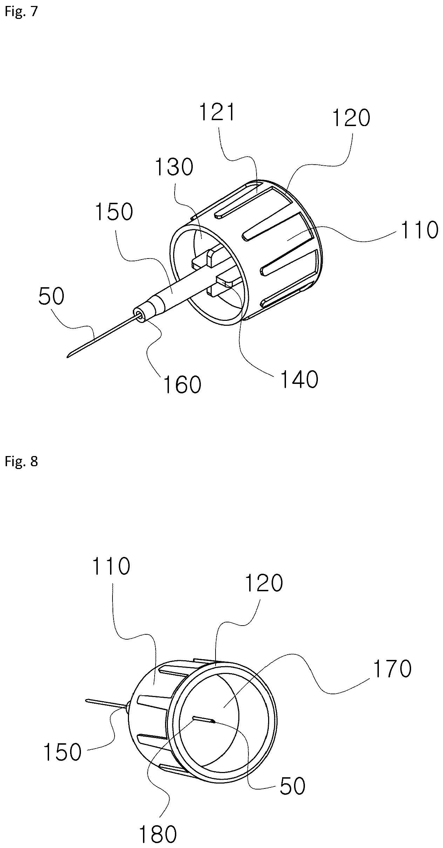

As illustrated in FIG. 7, a first flat portion 130 including a flat surface is included on the inner central portion of the first housing 110. A first support portion 140 and a second support portion 150 protrude from one side of the first flat portion 130.

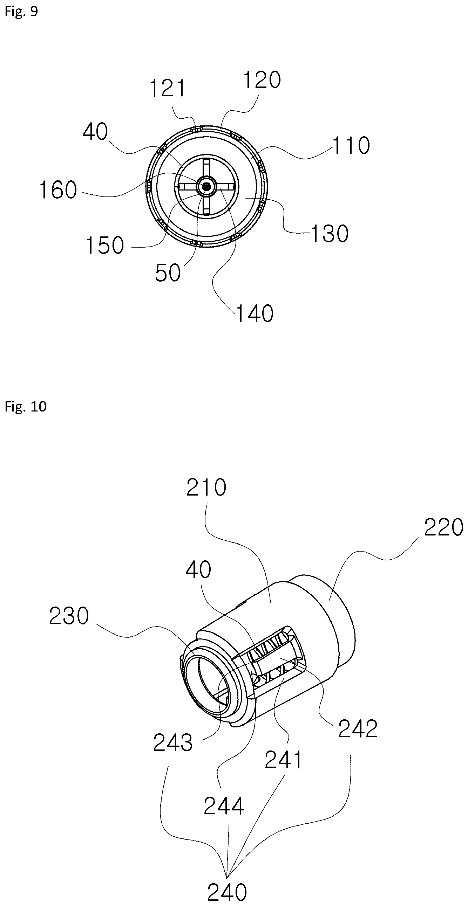

As illustrated in FIG. 9, the first support portion 140 is in the shape of a cross (+) when viewed from the front. The elastic member 40 may be fixed to the first support portion 140.

In addition, particularly, the diameter of the first support portion 140 may be set to be the same as the inner diameter of the elastic member 40. Thus, the elastic member 40 is prevented from being released from the first support portion 140 due to the tension and compression force of the elastic member 40, thereby minimizing the defective rate of the disposable safety syringe needle 1.

In particular, since the elastic member 40 is fixed to the first support portion 140, the tension and compression force of the elastic member 40 are directly transferred to the safety part 30, thereby making it impossible to reuse the disposable safety syringe needle 1 after use thereof. Advantageously, a third person is prevented from being infected by the used needle member.

As illustrated in FIG. 7, the second support portion 150 is formed as a hollow cylinder, such that the needle member 50 may be introduced into and fixed to the second support portion 150.

Here, as illustrated in FIGS. 7 and 8, the needle member 50 extends through the second support portion 150. Thus, particularly, the needle member 50 may be configured to be exposed to the outside from the second support portion 150.

In addition, a first hole 160 and a second hole 180 are formed in one end and the other end of the second support portion 150, respectively, as holes through which the needle member 50 is introduced.

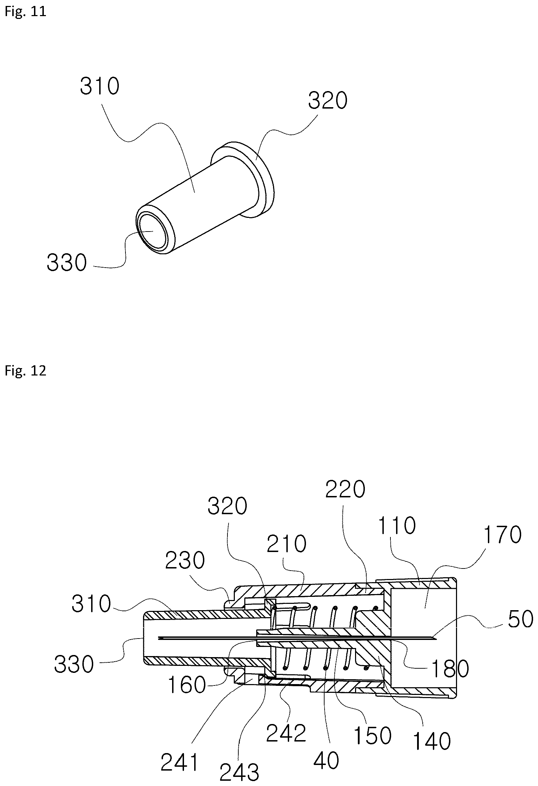

In addition, as illustrated in FIG. 12, the second support portion 150 is configured such that the inner diameter thereof gradually decreases in the direction from the first hole 160 to the second hole 180. Here, the inner diameter of the first hole 160 may be set to be greater than the outer diameter of the needle member 50, and the inner diameter of the second hole 180 may be set to be the same as the outer diameter of the needle member 50.

When the inner diameter of the first hole 160 is set to be smaller than or the same as the outer diameter of the needle member 50, the tip portion of the needle member 50 may be blunted during the introduction of the needle member 50 into the second support portion 150, so that the function of the needle member 50 as a needle may be lost.

In addition, when the inner diameter of the second hole 180 is greater than the outer diameter of the needle member 50, the needle member 50 may not be fixed to the second support portion 150, thereby causing a medical accident.

That is, the needle member 50 is fixed after being introduced in the direction from the first hole 160 to the second hole 180 in order to prevent a medical accident while not losing the function as a needle.

The first housing 110 includes a first guide 120 and first guide protrusions 121 on outer peripheral portions of the first housing 110 to prevent the disposable safety syringe needle 1 from slipping while being coupled to the syringe device 2.

As illustrated in FIG. 8, the first guide 120 may be formed on the outer peripheral portion of the other end of the 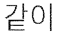 first housing 110, and the first guide protrusions 121 may protrude from one side of the first guide 120 while being equally spaced apart from each other.

Next, as illustrated in FIGS. 6 and 10, the bottom part 20 includes a second housing 210 configured to be coupled to one end of the first housing 110. The second housing 210 is in the shape of a hollow cylinder. One or more latch portions 240 are formed on outer peripheral portions of the second housing 210.

A third coupling portion 230 and a second coupling portion 220 protrude from one end and the other end of the second housing 210, respectively.

As illustrated in FIG. 12, the second coupling portion 220 is introduced into and connected to one end of the first housing 110.

As illustrated in FIG. 12, the other end of the safety part 30 to be described later is introduced into and connected to the third coupling portion 230.

As illustrated in FIG. 10, the latch portions 240 include a latch indicator 241, a third support portion 242, a latch stepped portion 243, and a latch recess 244.

As illustrated in FIG. 10, the latch indicator 241 is formed in the outer circumferential portion of the second housing 210 to be open like a window, through which whether or not the disposable safety syringe needle 1 is used may be visually identified.

As illustrated in FIG. 10, the third support portion 242 protrudes from one side of the latch indicator 241 to be parallel to the second housing 210. The outer periphery of a latch protrusion 320 of the safety part 30 introduced into the second housing 210 contacts the inner surface of the third support portion 242. Consequently, the safety part 30 may move vertically up and down along the inner surface of the third support portion 242 so as not to be released through the latch indicator 241.

As illustrated in FIGS. 10 and 12, the latch stepped portion 243 is provided on one end of the third support portion 242 to protrude into the second housing 210, thereby forming an inclined surface.

In particular, as illustrated in FIG. 12, at the initial step, the outer surface of the latch protrusion 320 of the safety part 30 contacts the inner protruding surface of the latch stepped portion 243. Thus, the latch stepped portion 243 may prevent the latch protrusion 320 from being moved toward the latch recess 244 and being fixed by the application of external force before the use of the disposable safety syringe needle 1.

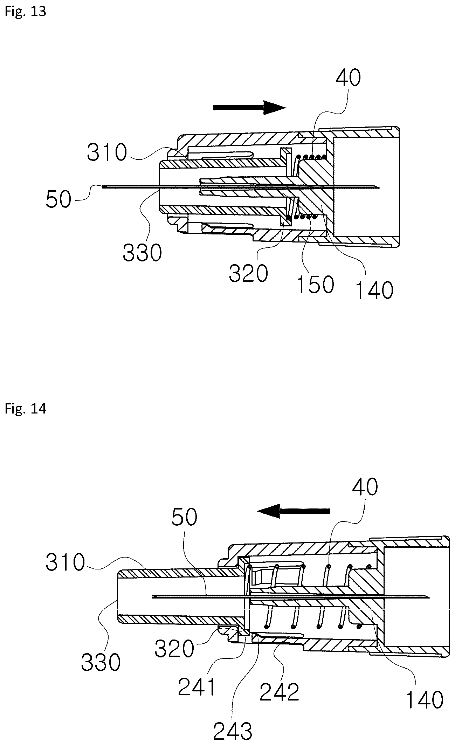

As illustrated in FIG. 13, during the use, the safety part 30 is introduced into the second housing 210, and the elastic member 40 is compressed. Afterwards, due to the tension (i.e., stretching force) of the elastic member 40, the outer surface of the latch protrusion 320 of the safety part 30 is accelerated along the inner inclined surface of the latch stepped portion 243 to move downward over the inner protruding surface of the latch stepped portion 243 so as to be latched by the latch recess 244 to be described later.

In particular, the disposable safety syringe needle 1 is configured such that the latch protrusion 320, which has moved down to the latch recess 244, is latched by the protruding surface of the latch stepped portion 243, thereby being prevented from moving up again. Thus, it is impossible to forcibly reuse the disposable safety syringe needle 1, so that puncture wounds or injuries that could otherwise be caused by the needle member can be advantageously prevented.

In addition, even when the user pushes the latch protrusion 320 latched by the latch recess 244 with a fingernail or a tool, the latch protrusion 320 latched by the protruding surface of the latch stepped portion 243 cannot move up again. Consequently, it is advantageously possible to prevent tampering by a user.

As illustrated in FIG. 10, the latch recess 244 is formed between the other side surface of the latch indicator 241 and one end of the third support portion 242. The latch protrusion 320 of the safety part 30 is latched and fixed due to the compression force and tension of the elastic member 40.

As illustrated in FIG. 10, one or more latch portions 240 may be formed at equal distances on outer peripheral portions of the second housing 210. For example, three latch portions 240 may be formed at equal distances on outer peripheral portions of the second housing 210, but the present disclosure is not limited thereto.

In particular, a plurality of latch portions 240 are formed at equal distances on outer peripheral portions of the second housing 210. At the initial step, the safety part 30 is balanced, and minute force of the free length of the elastic member 40 is endured. Thus, the safety part 30 is advantageously prevented from moving down toward the latch recess 244.

In addition, the plurality of latch portions 240 are formed at equal distances on the outer peripheral portions of the second housing 210, and the safety part 30 is introduced in the balanced position. Thus, a structure in which the latch protrusion 320 is moved over the latch stepped portion 243 to be locked to the latch recess 244 by the tension of the compressed elastic member 40 is advantageously formed.

When a single latch portion 240 is formed on an outer peripheral portion of the second housing 210, the safety part 30 may be introduced in an unbalanced position, so that the latch protrusion 320 may fail to move over the latch stepped portion 243. Consequently, a third person may be injured or infected by the needle member.

As illustrated in FIGS. 6 and 11, the safety part 30 is coupled to one end of the second housing 210, and includes a third housing 310 in the shape of a hollow cylinder and the latch protrusion 320 protruding from the other end of the third housing 310.

A third hole 330 is formed in one end of the third housing 310, and the latch protrusion 320 protrudes from the other end of the third housing 310.

In addition, the third housing 310 surrounds the needle member 50 therein, such that the needle member 50 is not exposed before use of the disposable safety syringe needle 1. Injuries that could be caused by the needle member are advantageously prevented.

As illustrated in FIG. 12, the latch protrusion 320 is introduced into and connected to one end of the second housing 210. In this structure, the outer surface of the latch protrusion 320 and the inner surface of one end of the second housing 210 are latched. Thus, the safety part 30 is prevented from being released from the second housing 210.

In addition, the inner surface of the latch protrusion 320 is coupled to the elastic member 40, such that the safety part 30 is movable vertically up and down. Thus, the structure by which the needle member 50 is concealed and exposed can prevent injuries and infections that could be caused by the needle member.

In particular, when the outer surface of the latch protrusion 320 is fixed by latching to the latch recess 244, the latch protrusion 320 cannot move up again, thereby preventing the disposable safety syringe needle 1 from being reused. Consequently, a medical accident that could be caused by the reuse of the disposable safety syringe needle 1 can be prevented.

The third hole 330 is a hole formed on one end of the third housing 310. During use of the disposable safety syringe needle 1, the needle member 50 is exposed from the third housing 310 through the third hole 330.

As illustrated in FIGS. 12 to 14, one end of the elastic member 40 is coupled to the outer surface of the first support portion 140 and the other end of the elastic member 40 is coupled to the inner surface of the latch protrusion 320, such that the safety part 30 is driven to move vertically up and down by the compression and tension of the elastic force. Thus, the structure capable of concealing and exposing the needle member 50 is provided.

As illustrated in FIG. 6, the elastic member 40 may be implemented as a spring elastically deformable by force applied thereto. Particularly, the elastic member 40 may be, but is not limited to, a compression spring.

The compression spring is a spring resisting to compression force. That is, since the compression spring is used, after the safety part 30 is introduced into the bottom part 20 in the direction of the top part 10 due to the compression, the latch protrusion 320 of the safety part 30 may be coupled to the one or more latch portions 240 due to the tension of the elastic member 40, thereby providing a non-reusable structure.

As illustrated in FIGS. 6 and 12, the needle member 50 is a metal tube, i.e., a syringe needle, obliquely recessed and extending through the second support portion 150.

In addition, the outer diameter of the needle member 50 may be set to range from 0.2 mm to 1.7 mm, but is not limited thereto. The outer diameter of the needle member 50 may vary depending on the intended use.

When the outer diameter of the needle member 50 is less than 0.2 mm, it is difficult to inject a medicine into a patient using the disposable safety syringe needle 1 although the patient is less painful. In particular, it is more difficult to inject a high viscosity medicine.

In addition, when the needle member 50 is formed slender, the needle member 50 is easily breakable, and thus the length thereof is limited.

When the outer diameter of the needle member 50 is greater than 1.7 mm, pain applied to the patient may be significantly increased, which is problematic.

In addition, the outer diameter of the needle member 50 may be categorized according to the intended use.

That is, when the needle member 50 is intended to be used for intravenous injection, the outer diameter of the needle member 50 may be set to range from 0.56 mm to 1.6 mm. When the needle member 50 is intended to be used for intramuscular injection, the outer diameter of the needle member 50 may be set to range from 0.6 mm to 0.86 mm.

In addition, when the needle member 50 is intended to be used for subcutaneous injection, the outer diameter of the needle member 50 may be set to range from 0.25 mm to 0. 5mm. When the needle member 50 is intended to be used for intradermal injection, the outer diameter of the needle member 50 may be set to range from 0.4 mm to 0.5 mm.

The disposable safety syringe needle 1 having the above-described configuration may be operated by a use method, as illustrated in FIGS. 12 to 14.

As illustrated in FIG. 12, a first step is before the disposable safety syringe needle 1 is used, wherein the outer surface of the latch protrusion 320 of the safety part 30 moving vertically up and down is in contact with the inner protruding surfaces of the plurality of latch stepped portions 243.

Here, in the first step, the needle member 50 exposed from the second support portion 150 is surrounded inside the third housing 310, so that the needle member 50 is not exposed to the outside from the safety part 30. Thus, it is possible to prevent injuries from being caused by the needle member.

Afterwards, as illustrated in FIG. 13, a second step is during use of the disposable safety syringe needle 1. In this step, the safety part 30 is introduced into the second housing 210, so that the first support portion 140 and the elastic member 40 provided on the latch protrusion 320 are compressed in the direction of the first housing 110.

In addition, in the second step, the outer peripheral portion of the latch protrusion 320 is introduced into the second housing 210 by a length by which the elastic member 40 is compressed.

Here, as the safety part 30 is introduced into the bottom part 20, the needle member 50 that has been within the third housing 310 is exposed to the outside. The needle member 50 does not move while being fixed to the second support portion 150.

Afterwards, as illustrated in FIG. 14, a third step is after use of the disposable safety syringe needle 1. As the elastic member 40 that has been compressed is stretched, the safety part 30 that has been within the bottom part 20 protrudes out from the bottom part 20.

That is, the needle member 50 that has been exposed is surrounded again by the third housing 310, thereby being concealed in the safety part 30.

Due to the tension of the elastic member 40, the plurality of third support portions 242 prevent the outer surface of the latch protrusion 320 from being released through the latch indicator 241.

In addition, the outer surface of the latch protrusion 320 is accelerated along the inner inclined surfaces of the plurality of latch stepped portions 243. Due to the tension and acceleration, the outer surface of the latch protrusion 320 moves over the inner protruding surfaces of the plurality of latch stepped portions 243, thereby being latched by the latch recess 244.

In particular, the latch protrusion 320 is configured such that the outer peripheral portion thereof does not contact the inner surface of the second housing 210 so as not to generate friction. Thus, when the outer surface of the latch protrusion 320 moves vertically up and down along the inner inclined surfaces of the plurality of latch stepped portions 243, the outer surface of the latch protrusion 320 may be advantageously accelerated.

Furthermore, as the latch protrusion 320 latched by the plurality of latch recesses 244 is also latched by the protruding surface of the latch stepped portion 243, the latch protrusion 320 cannot move up again. Thus, the disposable safety syringe needle 1 cannot be reused, and the needle member 50 is not exposed from the safety part 30.

That is, since the needle member 50 is not exposed, a third person can advantageously be prevented from being injured or infected by the needle member 50 after use.

In addition, due to the structure preventing the safety part 30 from moving up again, tampering by a user can be prevented, thereby fundamentally preventing reuse. Accordingly, infection caused by reuse as well as infection caused by viruses, such as the AIDS virus, or bacteria can be prevented.

### MODE FOR INVENTION

### <Second Embodiment>

A disposable safety syringe needle 1, the same as that of the first embodiment as described above, may further include a sealing part 60.

The disposable safety syringe needle 1 is configured the same as that of the first embodiment, and thus a description thereof will be omitted.

The sealing part 60 may be removed to prevent the sterilized disposable safety syringe needle 1 from being contaminated before transportation or use thereof, and be formed of a synthetic resin or paper.

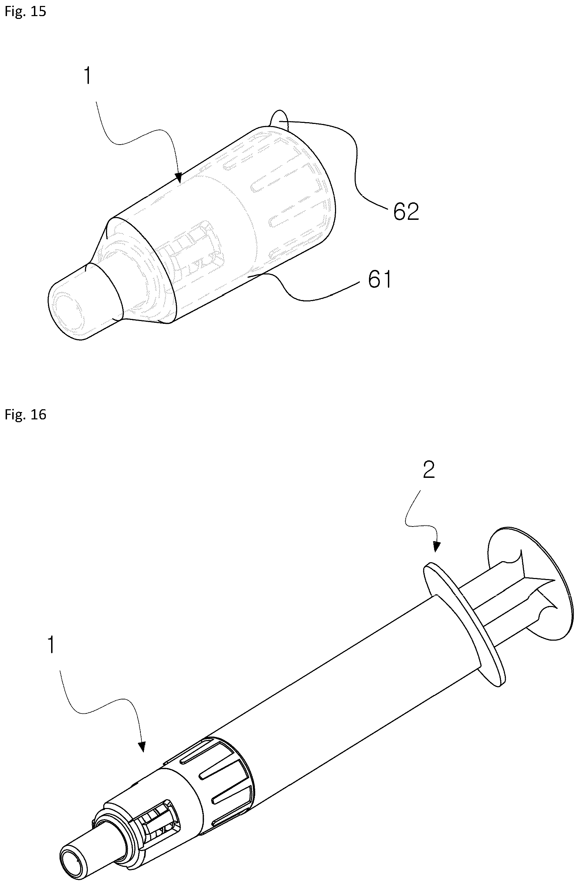

As illustrated in FIG. 15, the sealing part 60 includes a first sealing portion 61 and a second sealing portion 62.

As illustrated in FIG. 15, one end of the first sealing portion 61 is closed and the other end of the first sealing portion 61 is open. The first sealing portion 61 may be manufactured by plastic injection to protect the outer portion of the disposable safety syringe needle 1, and formed of, but is not limited to, PVC, PE, or PP.

In addition, before the sealing part 60 is removed, the first sealing portion 61 may be manufactured to be transparent so that a user may easily identify the state of the disposable safety syringe needle 1 from the outer appearance of the first sealing portion 61.

The outer surface of the safety part 30 is positioned inside one end of the first sealing portion 61, and the outer surface of the top part 10 is positioned inside the other end of the first sealing portion 61.

In addition, a plurality of first anti-slip portions (not shown) are formed at equal distances on the outer peripheral surface of the first sealing portion 61. When the second sealing portion 62 is removed, it is advantageously possible to prevent the first sealing portion 61 from slipping away from the hand of the user.

Furthermore, a plurality of second anti-slip portions (not shown) may be formed at equal distances on the inner surface of the other end of the first sealing portion 61.

The first anti-slip portions and the second anti-slip portions may be formed in the same shape as the first guide protrusions 121 as illustrated in FIG. 7, but the present disclosure is not limited thereto.

The second anti-slip portions are fitted to recesses alternating with the first guide protrusions 121 formed at equal distances, so that there is an advantage in that the disposable safety syringe needle 1 is immovably fixed within the sealing part 60.

The second sealing portion 62 is formed on the other end of the first sealing portion 61 to prevent the disposable safety syringe needle 1 from being contaminated. The second sealing portion 62 may be formed of sterilized paper which is removable.

Since the sealing part 60 is further included in the disposable safety syringe needle 1, a step of removing the sealing part 60 may be performed before use of the disposable safety syringe needle 1.

### <Third Embodiment>

A syringe device 2 may include the disposable safety syringe needle 1 according to the first embodiment or the second embodiment.

The disposable safety syringe needle 1 is configured the same as that of the first embodiment or the second embodiment, and thus a description thereof will be omitted.

The syringe device 2 may include a syringe or an automatic syringe.

The syringe may be implemented as a well-known syringe. For example, as illustrated in FIG. 16, the syringe may include, but is not limited to, an outer barrel, a barrel flange, a plunger, a rubber packing, and a plunger flange.

When the disposable safety syringe needle 1 is coupled to the syringe, a user (i.e., a medical personnel) can be advantageously relieved from the stress of being pricked by the needle.

In addition, when the syringe including the disposable safety syringe needle 1 is discarded, a person carrying the syringe can be advantageously prevented from being injured or infected by being pricked by the used needle.

The automatic syringe is a syringe designed to inject a medicine. The automatic syringe may be categorized as a pump type syringe or a pen type syringe, and inject a medicine once or several times.

In a situation in which the disposable safety syringe needle 1 is coupled to the automatic syringe, when the syringe is discarded, the needle member 50 is not exposed even if the syringe is not covered with a separate safety cap. Thus, there are advantages in that no injuries occur and a discard process is convenient.

The medicine may include one or more selected from, but is not limited to, insulin, hormone, low-molecular-weight heparin, or derivatives thereof.

A method of coupling the disposable safety syringe needle 1 and the syringe device 2 may include introducing a needle-coupling portion of the syringe device 2 into the other end of the first housing 110 of the disposable safety syringe needle 1.

### INDUSTRIAL APPLICABILITY

The present disclosure relates to a disposable safety syringe needle configured to prevent reuse thereof as well as accidents in which the needle causes injury and a syringe device including the same. Accordingly, the present disclosure is industrially applicable.

## Claims

1. A disposable safety syringe needle comprising:
a top part (10) configured such that an elastic member (40) is coupled and fixed to the top part (10) and a needle member (50) is introduced into and fixed to the top part (10);
a bottom part (20) coupled to one end of the top part (10), and comprising one or more latch portions (240); and
a safety part (30) coupled to one end of the bottom part (20) and comprising a latch protrusion (320),
wherein, after the safety part (30) is introduced into the bottom part (20) in a direction of the top part (10) so that the elastic member (40) is compressed, the latch protrusion (320) of the safety part (30) is coupled to the one or more latch portions (240) due to tension of the elastic member (40), thereby making reuse of the disposable safety syringe needle impossible.

2. The disposable safety syringe needle of claim 1, wherein the top part (10) comprises:
a first housing (110) with open one and the other ends;
a first flat portion (130) provided on an inner portion of the first housing (110) and comprising a flat surface; and
a first support portion (140) and a second support portion (150) protruding from one side of the first flat portion (130) .

3. The disposable safety syringe needle of claim 2, wherein the top part (10) further comprises a first guide (120) and a first guide protrusions (121) on outer peripheral portions of the first housing (110) to prevent the disposable safety syringe needle from slipping during use.

4. The disposable safety syringe needle of claim 2, wherein the second support portion (150) comprising the open one and the other ends is configured such that a diameter thereof decreases from one end to the other end thereof.

5. The disposable safety syringe needle of claim 1, wherein the latch portions (240) comprise:
a latch indicator (241) provided in an outer circumferential portion of a second housing (210) to allow whether or not the disposable safety syringe needle is used to be identified;
a third support portion (242) protruding from one side of the latch indicator (241);
a latch stepped portion (243) provided on one end of the third support portion (242), protruding into the second housing (210), and being inclined; and
a latch recess (244) provided between the latch indicator (241) and the third support portion (242), such that the latch protrusion (320) of the safety part (30) is fixed by compression force of the elastic member (40).

6. The disposable safety syringe needle of claim 1, further comprising a sealing part (60) to prevent the sterilized disposable safety syringe needle from being contaminated, wherein the sealing part (60) is removable from the disposable safety syringe needle.

7. A syringe device including a disposable safety syringe needle as claimed in any one of claims 1 to 6.
